# EUROPEAN PATENT SPECIFICATION

(11) **EP 2 554 140 B1**
(45) Date of publication and mention of the grant of the patent: **19.04.2017**
(21) Application number: 11762582.2
(22) Date of filing: 16.03.2011
(51) Int. Cl.: A61F 2/82, A61F 2/04

(54) **STENT**
STENT
STENT

(30) Priority: 30.03.2010 JP 2010076616
(43) Date of publication of application: 06.02.2013
(73) Proprietor: Terumo Kabushiki Kaisha, Tokyo 151-0072 (JP)
(72) Inventor: UCHIYAMA, Sayaka, Ashigarakami-gun Kanagawa 259-0151 (JP); SAITO, Noboru, Ashigarakami-gun Kanagawa 259-0151 (JP); NAGURA, Hiroaki, Ashigarakami-gun Kanagawa 259-0151 (JP)
(74) Representative: Dossmann, Gérard
(86) International application number: PCT/JP2011/056219
(87) International publication number: WO 2011/122344

(56) References cited:
- WO-A1-03/047463
- WO-A2-01/93781
- JP-A- 2002 095 756
- JP-A- 2005 118 123
- JP-A- 2008 200 492
- US-A1- 2007 112 421

## Description

### Technical Field

The present invention relates to a stent which is set indwelling in a lesion part such as a stenosed part or an occluded part generated in a lumen in a living body, so as to maintain patency of the lesion part.

### Background Art

Conventionally, the stent indwelling technique has been conducted wherein a stent, a hollow tubular medical device, is set indwelling in a lesion part such as a stenosed part or an occluded part generated in a lumen or a body cavity in a living body such as blood vessel, bile duct, esophagus, trachea, urethra or other organ, so as to maintain patency of the lesion part.

US2007112421 relates generally to medical devices, such as stents, for providing a medical treatment to an area of a patient, such as a body lumen. More particularly, the document is directed to a stent comprising a grooved surface. Moreover, a coating may be applied to a strut in a variety of thicknesses, layers, and/or patterns.

For instance, percutaneous transluminal coronary angioplasty (PTCA) for treating ischemic heart disease is a technique which includes the steps of minutely cutting an artery in a patient's leg or arm, placing an introducer sheath (introducing device) there, inserting a long hollow tube called guide catheter into the blood vessel through the lumen of the introducer sheath, with a guide wire being let precede, and placing the guide catheter at the entrance to a coronary artery. In the technique, thereafter, the guide wire is pulled out, a balloon catheter with another thin guide wire inserted therein is inserted into the lumen of the guide catheter, the balloon catheter is advanced to a lesion part (stenosed part or occluded part) in the patient's coronary artery under radioscopy, while letting the guide wire precede, the balloon is positioned in the lesion part, and the balloon is inflated in the part once or plural times at a predetermined pressure by the surgeon. By this technique, the lumen of the blood vessel in the lesion part is dilated and maintained patency, whereby the bloodstream through the vascular lumen is increased. It has been reported, however, that when the vascular wall is injured by the catheter, vascular intimal proliferation as a curative reaction of the vascular wall occurs, leading to high possibility of restenosis.

In consideration of this problem, in order to lower the restenosis rate, the stent indwelling technique has been practiced in which a stent is put indwelling in the lesion part having been dilated by the balloon. Although the stenosis rate has been lowered by the stent indwelling technique, however, perfect prevention of restenosis has not yet been attained.

In view of this, in recent years, there have vigorously been proposed various attempts to lower the restenosis rate by use of a drug-eluting stent (DES), that is, a stent with a biologically active agent such as immunosuppressant or carcinostatic agent loaded thereon. In this case, the biologically active agent is sustainedly released in the lesion part where the stent is put indwelling, thereby suppressing the vascular intimal proliferation, which is considered to the cause of restenosis. By the advent of the DES, the restenosis rate has been lowered remarkably.

On the other hand, as a problem involved in DES, it has recently been reported that late stent thrombosis is liable to occur in the lumen of the stent put indwelling in the lesion part, probably because of delay of coating with vascular endothelial cells.

In order to solve such a problem, for example, Patent Document 1 proposes a stent wherein a substance having a cell adhesion ability to promote adhesion of vascular endothelial cells is provided in the state of solid phase on an inner surface of the stent. This stent is aimed at restraining of late stent thrombosis and/or restenosis by causing growth of vascular endothelial cells on the inner surface of the stent.

### Prior Art Document

### Patent Document

Patent Document 1: Japanese Patent Laid-Open No. 2005-118123

### Summary of Invention

The invention relates to a device as defined by claim 1 or claim 2. Insofar as the terms "invention" and/or "embodiment" are used in the following, and/or features are presented as being optional, this should be interpreted in such a way that the only protection sought is that of the invention as claimed.

Meanwhile, for the vascular endothelial cells, in general, a spindle-like shape with a major axis along the direction of blood flow is said to be a mature shape, and, by assuming such a spindle-like shape, the endothelial cells become functional cells. In the stent described in the above-mentioned Patent Document 1, however, the substance having the cell adhesion ability is provided on substantially the whole area of the inner surface of the stent, so that vascular endothelial cells deposited thereon tend to grow at random, and it is difficult for the endothelial cells to take a spindle-like shape.

The present invention has been made in consideration of the above-mentioned problems. Accordingly, it is an object of the present invention to provide a stent wherein cells are directionally proliferated on the inner surface of the stent and thus the stent surface is quickly coated with the cells so that the onset of late stent thrombosis or restenosis can be restrained.

In order to attain the above object, according to the present invention, there is provided a stent including: a cylindrical stent body which has openings at both ends and extends along a longitudinal direction between the openings at the both ends; and a coating layer which is provided on an inner surface of the stent body and contains a substance having a cell adhesion ability to promote adhesion of cells, wherein the coating layer is formed by arranging a plurality of linear coating parts, each extending linearly, in a striped pattern, in the way as described in claim 1 or claim 2.

In the stent according to the present invention which is configured as above-mentioned, the coating layer on the inner surface of the stent body is formed by arranging a plurality of the linear coating parts, each extending linearly and having the cell adhesion property, in the striped pattern. This makes it possible that the cells can be made to grow on the inner surface of the stent main body, with a desirable directionality. As a result, growth of the cells is promoted, and the stent surface is quickly coated with the cells. In addition, the onset of late stent thrombosis or stenosis can be restrained.

In the case where the linear coating parts are so provided as to extend along the longitudinal direction of the stent body when the stent body is expanded, the cells can be proliferated on the linear coating parts, directionally along the longitudinal direction of the stent body. Especially, for vascular endothelial cells, a spindle-like shape with a major axis along the direction of blood flow is said to be a mature shape, and, by assuming such a spindle-like shape, the endothelial cells become functional cells. In connection with this fact, the coincidence of the blood flow direction and the extending direction of the linear coating parts enables the endothelial cells to be grown in the functional spindle-like shape having the major axis along the longitudinal direction of the stent body.

In the case where the linear coating parts are so provided as to extend along the circumferential direction of the stent body when the stent body is expanded, the cells can be proliferated on the linear coating parts, directionally along the circumferential direction of the stent body. Particularly, vascular smooth muscle cells are arranged helically to constitute the blood vessel. In connection with this fact, since the helix is wound along the circumferential direction of the stent body, the direction of the vascular smooth muscle cells coincide roughly with the extending direction of the liner coating parts. Accordingly, the smooth muscle cells can be grown to be arranged along the circumferential direction of the stent body.

In the case where the liner coating parts are provided in the state of filling grooves formed in the inner surface of the stent body, the coating layer can be restrained from being broken at the time of an operation of mounting the stent onto a balloon or a balloon-inflating operation.

In the case where the substance having the cell adhesion ability is a synthetic peptide, cells can be adhered favorably.

### Brief Description of Drawings

[FIG. 1]

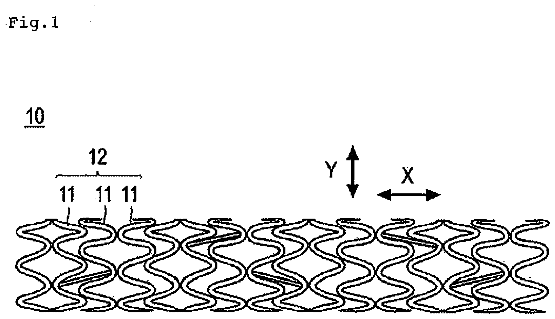

   FIG. 1 is a perspective view showing a stent according to a first embodiment.
[FIG. 2]

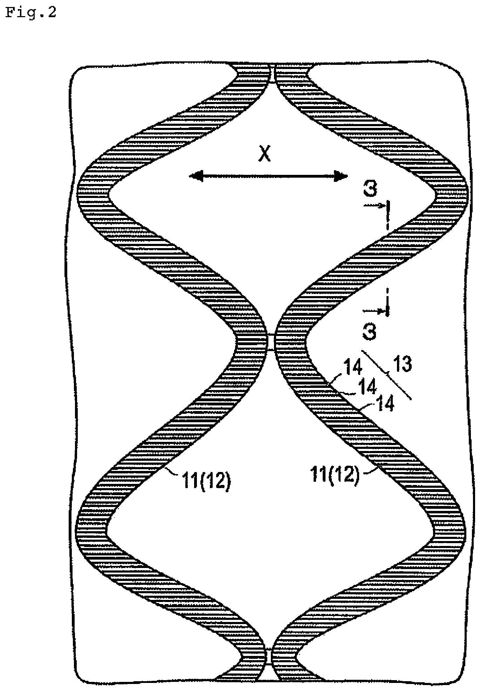

   FIG. 2 is a partially enlarged plan view showing an inner surface of the stent according to the first embodiment.
[FIG. 3]

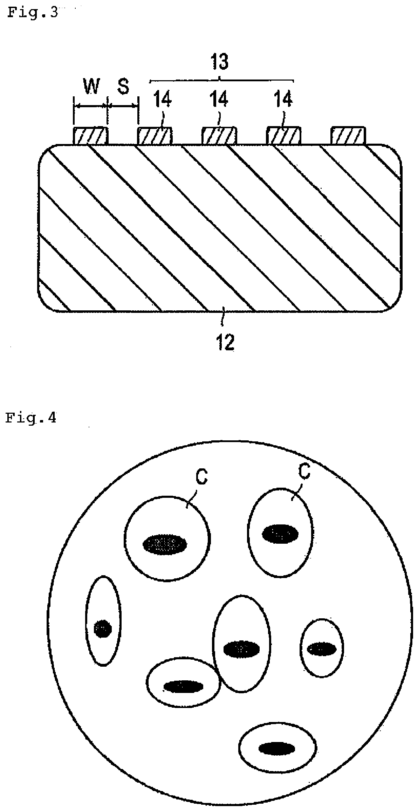

   FIG. 3 is a sectional view taken along line 3-3 of FIG. 2.
[FIG. 4]
   FIG. 4 is a schematic view illustrating an example of proliferation of cells in the case where a coating layer is provided in a plane surface form on a stent body.
[FIG. 5]

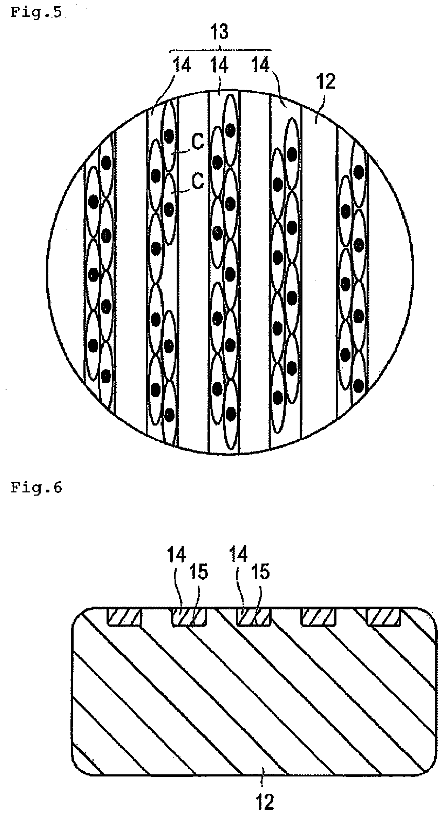

   FIG. 5 is a schematic view illustrating an example of proliferation of cells in the stent according to the first embodiment.
[FIG. 6]
   FIG. 6 is a sectional view showing a modification of the stent according to the first embodiment.
[FIG. 7]

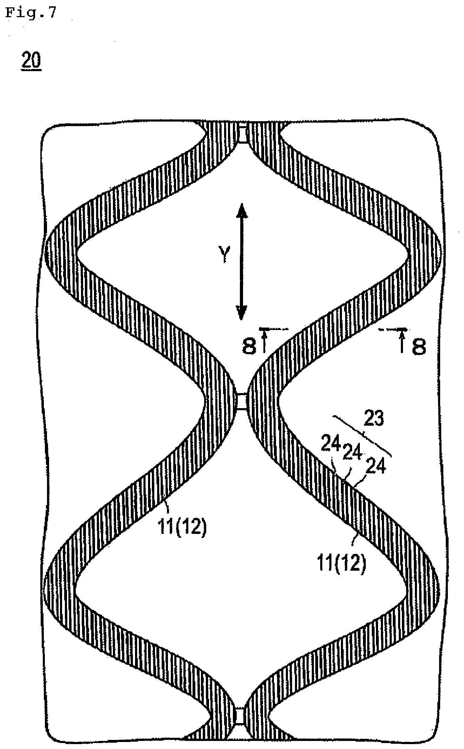

   FIG. 7 is a partially enlarged plan view showing an inner surface of a stent according to a second embodiment of the present invention.
[FIG. 8]

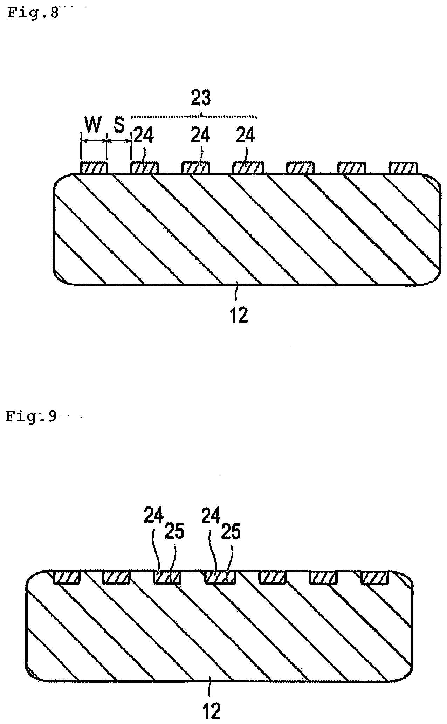

   FIG. 8 is a sectional view taken along line 8-8 of FIG. 2.
[FIG. 9]
   FIG. 9 is a sectional view showing a modification of the stent according to a second embodiment.
[FIG. 10]

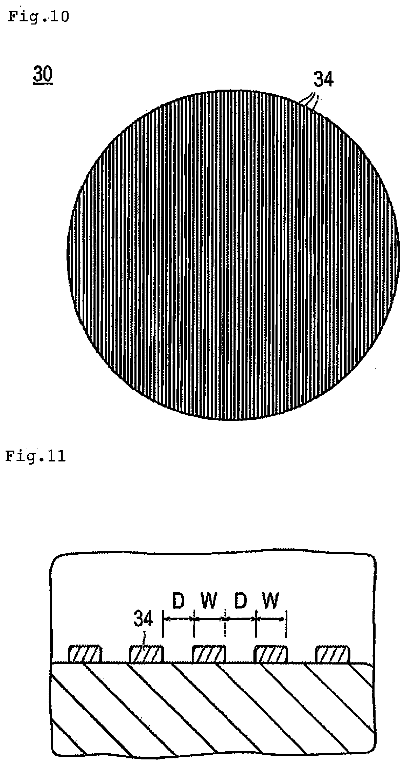

   FIG. 10 is a plan view showing a part of a coated substrate according to a reference example.
[FIG. 11]
   FIG. 11 is a sectional view showing a part of the coated substrate according to the reference example.
[FIG. 12]

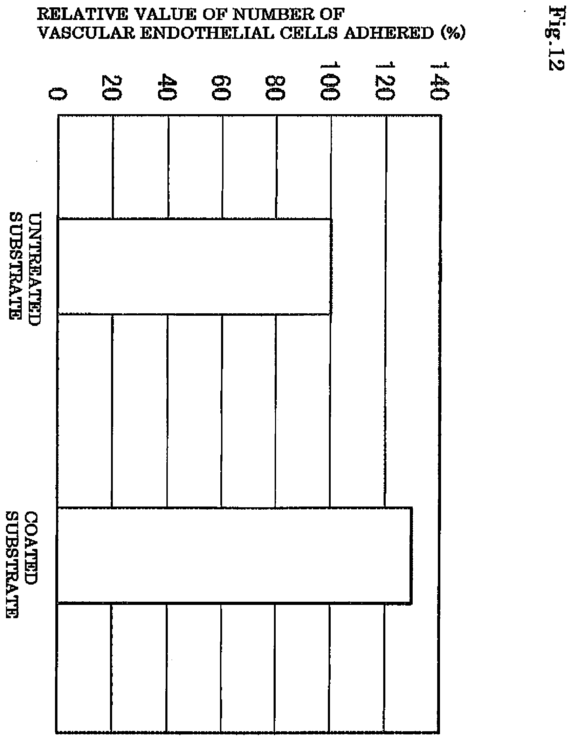

   FIG. 12 is a graph showing the numbers of vascular endothelial cells adhered to an untreated substrate and the coated substrate according to the reference example.

### Mode for Carrying Out the Invention

Now, embodiments of the present invention will be described below, referring to the drawings. Incidentally, the dimensional ratios in the drawings may be exaggerated and be different from actual ratios, for convenience of illustration.

### <First Embodiment>

As shown in FIGS. 1 to 3, a stent 10 according to a first embodiment of the present invention includes a tubular stent body 12 wherein a plurality of annular parts 11 composed of linear material bent in a wavy form and formed in an annular shape are arranged in an axial direction and interconnected, and a coating layer 13 provided on an inner surface of each of the annular parts 11. The stent 10 is of a balloon-expandable type, and is placed on a balloon of a balloon catheter, wherein the stent body 12 is radially expanded by inflating the balloon. When the stent body 12 is expanded, it is plastically deformed so that the bend angle of each of the annular parts 11 is widened, and the stent 10 is put indwelling in a lesion part while maintaining a living body tissue or the like in the state of being expanded by the outer surface of the stent body 12. Here, the outer surface of the stent body 12 that makes contact with the living body tissue means a surface on the side of making contact with a living body tissue forming a lesion part, specifically, for example, an inner wall of a blood vessel or the like, when the stent 10 is put indwelling in the lesion part. On the other hand, the inner surface of the stent body 12 means a surface on the side of making contact with a body fluid, such as blood, when the stent 10 is put indwelling in a lesion part.

A coating layer 13 containing a substance having a cell adhesion ability to promote adhesion of cells is provided in the state of solid phase on the inner surface of the stent body 12. The coating layer 13 is for ensuring that, when the stent body 12 is expanded and put indwelling in a lesion part, the inner surface of the stent body 12 will be coated with vascular endothelial cells.

As shown in FIGS. 2 and 3, the coating layer 13 is formed in a striped pattern wherein a plurality of linear coating parts 14, each extending linearly, are arranged. The linear coating parts 14 are so provided as to extend along the longitudinal direction X of the stent body 12 in the state where the stent body 12 is put indwelling in a lesion part by being expanded by a balloon or the like. In other words, the linear coating parts 14 are made to coat the inner surface of the stent body 12, in consideration of the anticipated expanded state. Incidentally, the expanded state varies depending on the situation in which the stent 10 is used; therefore, the linear coating parts 14 in the expanded state of the stent body 12 may not necessarily be utterly parallel to the longitudinal direction X of the stent body 12. In addition, the linear coating parts 14 may not necessarily be parallel to one another. Furthermore, the linear coating parts 14 may not necessarily be rectilinear, and may be provided in a curved line form.

The coating layer 13 is formed in the state of solid phase on the inner surface of the stent body 12. This ensures that the coating layer 13 formed on the stent surface on the side of making contact with the balloon can be prevented from being broken at the time of an operation of mounting the stent 10 onto the balloon or a balloon-inflating operation.

Incidentally, in the present invention, the stent body 12 is not restricted to the mode as shown in the drawings, and may be in other mode insofar as it is a tubular body which can be expanded radially. The sectional shape of the linear material constituting the stent main 12 also is not restricted to the rectangular shape as shown in FIG. 3, and may be other shape such as circular shape, elliptic shape, and other polygonal shapes than rectangular shape.

In addition, the means for expanding the stent 10 is not particularly restricted to the balloon-expandable type; for example, the stent 10 may be of a self-expandable type, namely, a stent wherein when a force for holding a stent in a radially reduced folded state is removed, the stent expands radially by its own restoring force.

Examples of the material for the stent body 12 include polymer materials, metallic materials, carbon fiber, and ceramics. The material is not particularly restricted insofar as it has a certain extent of rigidity and elasticity, but it is preferable for the material to be a biocompatible material. Specific examples of the polymer materials include polyolefins such as polyethylene, polypropylene, etc., polyesters such as polyethylene terephthalate, etc., cellulose polymers such as cellulose acetate, cellulose nitrate, etc., and fluorine-containing polymers such as polytetrafluoroethylene, tetrafluoroethylene-ethylene copolymer, etc. Examples of the metallic materials include stainless steels, tantalum, titanium, nickel-titanium alloy, tantalum-titanium alloy, nickel-aluminum alloy, inconel, gold, platinum, iridium, tungsten, and cobalt alloys such as cobalt-chromium alloy. Among stainless steels, preferred is SUS316L, which is the best in corrosion resistance.

The stent body 12 can be favorably formed from a material that is suitably selected from the above-mentioned materials according to the application site and the expanding means. For instance, in the case where the stent body 12 is formed from a metallic material, the stent 10 can be assuredly put indwelling in a lesion part, since the metallic material is excellent in strength. Besides, in the case where the stent main 12 is formed from a polymer material, it exhibits an excellent effect as to deliverability of the stent 10 to the lesion part, since the polymer material is excellent in flexibility.

In addition, in the case where the stent 10 is of the self-expandable type, a superelastic alloy such as nickel-titanium alloy or the like is preferred as the material, since a restoring force for returning into an original shape is required. In the case where the stent 10 is of the balloon-expandable type, stainless steel or cobalt-chromium alloy or the like is preferred as the material, since it is preferable that shape restoration after expansion is not liable to occur.

Besides, in the case where the stent body 12 is formed from carbon fiber, excellent effects can be exhibited in terms of high strength, excellent flexibility, and high safety in living bodies.

The size of the stent body 12 may be appropriately selected according to the site (part) to which the stent 10 is to be applied. For example, where the stent 10 is to be used in a coronary artery, normally, the outside diameter of the stent body 12 before expansion is preferably 1.0 to 3.0 mm, and the length is preferably 5 to 50 mm.

The method for manufacturing the stent body 12 is not particularly limited, and may be appropriately selected from the ordinarily used manufacturing methods, according to the structure and material of the stent 10.

Examples of the substance having the cell adhesion ability that is contained in the coating layer 13 include polypeptides such as poly-L-arginine, etc.; synthetic polypeptides such as arginine (Arg)-glycine (Gly)-aspartic acid (Asp) (RGD) and BD^{™} PuraMatrix^{™} Peptide Hydrogel (registered trademark) having arginine, alanine and aspartic acid as three constituents, etc., which has a cell adhesion ability; synthetic substances that have a group having an ability to adsorb protein (e.g, albumin), such as octadecyl group or oleyl group.

The thickness of the coating layer 13, which depends on the shape and size of the stent 10 and the kind of the substance to be provided in the state of solid phase, is preferably set in such a range as to ensure that the effect of the provision of the substance in the state of solid phase is displayed sufficiently when the stent 10 is put indwelling in a lesion part, that the coating layer 13 would not be broken at the time of an operation of mounting the stent 10 onto a balloon or a balloon-inflating operation, and that a sufficient caulking force for caulking the stent 10 onto the balloon can be obtained. Specifically, the thickness of the coating layer 13 is preferably not more than 3 µm, more preferably not more than 2 µm, and further preferably not more than 1 µm. When the thickness of the coating layer 13 is not more than 3 µm, the coating layer 13 would not be broken at the time of the operation of mounting the stent 10 onto a balloon or the balloon-inflating operation, and a sufficient caulking force for caulking the stent 10 onto the balloon can be obtained.

The width W of each of the linear coating parts 14 is preferably such a size that vascular endothelial cells adhered thereto are liable to take a spindle-like shape. As for the shape of the vascular endothelial cells in a blood vessel, a spindle-like shape with a major axis along the direction of blood flow is said to be a manure shape, and, by taking such a spindle-like shape, the endothelial cells become functional cells. Therefore, when the width W of each of the linear coating parts 14 is such a size that the vascular endothelial cells adhered thereto are liable to assume a spindle-like shape, functional vascular endothelial cells similar to the intrinsic vascular endothelial cells can be proliferated. The width W of each of the linear coating parts 14 is preferably not more than 50 µm, more preferably not more than 25 µm, and further preferably not more than 12.5 µm.

The spacing S between the adjacent linear coating parts 14 is preferably such a size that the proliferated vascular endothelial cells are liable to take a spindle-like shape. Between the adjacent linear coating parts 14, the vascular endothelial cells are proliferated, after the proliferation of the vascular endothelial cells on the linear coating parts 14. Therefore, when the spacing S between the adjacent linear coating parts 14 is such a size that the vascular endothelial cells are liable to assume a spindle-like shape, functional vascular endothelial cells similar to the intrinsic vascular endothelial cells can be proliferated. The spacing S between the adjacent linear coating parts 14 is preferably not more than 50 µm, more preferably not more than 25 µm, and further preferably not more than 12.5 µm.

A method of manufacturing the stent 10 according to this embodiment as above-described will be described below.

First, a stent body 12 is prepared, and the substance having the cell adhesion ability is provided in the state of solid phase on an inner surface of the stent body 12. The method for providing the substance having the cell adhesion ability in the state of solid phase is not particularly limited, insofar as the substance can present in the state of solid phase on the surface of the stent body 12 so that the coating layer 13 would not be released from the stent body when the stent 10 is put indwelling in a lesion part. Therefore, the coating layer 13 may be provided in the state of solid phase on the surface of the stent body 12 by covalent bond, or the coating layer 13 may be provided in the state of solid phase on the surface of the stent body 12 by ionic bond. Furthermore, a method wherein the substance having the cell adhesion ability is applied in a striped pattern on the surface of the stent body 12 by coating or the substance having the cell adhesion ability is applied by spraying or dipping after making the surface in a striped pattern and thereafter the coating layer 13 is solidified by drying or a heat treatment to provide the substance in the state of solid phase in a striped pattern, may be adopted insofar as the coating layer 13 can be prevented from being released from the stent body 12 when the stent 10 is put indwelling in a lesion part.

According to the stent 10 pertaining to this embodiment, the plurality of linear coating parts 14 containing the substance having the cell adhesion ability are formed to be arranged in a striped pattern on the inner surface of the stent body 12. Therefore, cells can be adhered directionally by the liner coating parts 14 which extend linearly when the stent 10 is expanded in the lesion part. As a result, the cells are adhered with a desired directionality, whereby the growth of the cells is promoted and the surface of the stent 10 is quickly coated with the cells, so that the onset of late stent thrombosis or restenosis can be restrained.

Particularly, for vascular endothelial cells in a blood vessel, a spindle-like shape with a major axis along the direction of blood flow is said to be a mature shape, and, by assuming such a spindle-like shape, the endothelial cells become functional cells. Then, in the case where for example the coating layer 13 is not formed in linear shapes but formed in a plane surface shape (for example, formed on the whole area of the inner surface of the stent body), as shown in FIG. 4, vascular endothelial cells C are proliferated at random, and are not liable to take a spindle-like shape. On the other hand, in the stent 10 according to this embodiment, the linear coating layers 14 having the cell adhesion ability are so provided as to extend along the longitudinal direction X of the stent body 12 when the stent body 12 is expanded. As shown in FIG. 5, therefore, the vascular endothelial cells C tend to be proliferated on the linear coating parts 14 and to grow in the functional spindle-like shape with the major axis along the longitudinal direction X. After proliferated on the linear coating parts 14, the vascular endothelial cells C are further proliferated on the stent body 12 in such a manner as to fill up the gaps between the adjacent linear coating parts 14, so that finally the whole part (or a part) of the inner surface of the stent body 12 is coated with the vascular endothelial cells C. Thus, by preliminarily limiting the ranges in which the vascular endothelial cells C are proliferated, the vascular endothelial cells C can be proliferated in a functional and desirable form similar to the intrinsic form thereof. Accordingly, quicker cell restoration can be realized, and the onset of late stent thrombosis or restenosis can be restrained.

Incidentally, while the substance having the cell adhesion ability is not provided between the adjacent linear coating parts 14 in this embodiment, this configuration is not restrictive. For example, a layer of a substance having a cell adhesion ability weaker than that of the linear coating parts 14 may be provided between the adjacent linear coating parts 14. Where such a configuration is adopted, at the time of proliferation of the vascular endothelial cells so as to fill up the gaps between the adjacent linear coating parts 14 after their proliferation on the linear coating parts 14, quicker proliferation of the cells can be realized.

In addition, as shown in FIG. 6, which illustrates a modification of the stent 10 according to the first embodiment, the linear coating parts 14 may be formed in such a manner as to fill up a plurality of grooves 15 formed in the inner surface of the stent body 12. This configuration ensures that the coating layer 13 can be more securely restrained from being broken at the time of an operation of mounting the stent onto a balloon or a balloon-inflating operation. Besides, this configuration ensures that the risk of breakage of the coating layer 13 can be lowered, without providing the linear coating parts 14 in the state of solid phase on the stent body 12.

### <Second Embodiment>

As shown in FIGS. 7 and 8, a stent 20 according to a second embodiment of the present invention differs from the stent 10 of the first embodiment in the direction in which linear coating parts 24 extend. Incidentally, the parts having the same functions as those of the parts in the first embodiment above will be denoted by the same reference signs as used above, and descriptions of the same parts will be omitted, for avoiding duplication.

Like in the first embodiment, a coating layer 23 containing the substance having the cell adhesion ability to promote adhesion of cells is provided in the state of solid phase on the inner surface of a stent body 12. The coating layer 23 is provided for ensuring that the inner surface of the stent body 12 will be coated with vascular smooth muscle cells after the stent body 12 is put indwelling in a lesion part by being expanded.

In general, vascular smooth muscle cells are arranged helically to constitute a blood vessel. In order to realize as better similarity as possible to the direction in which vascular smooth muscle cells are arranged, therefore, liner coating parts 24 are provided so as to extend along the circumferential direction Y of the stent body 12 in the state where the stent body 12 is put indwelling in a lesion part by being expanded by a balloon or the like. Incidentally, while the vascular smooth muscle cells are arranged helically to constitute a blood vessel, the helical pitch in a larger-diameter blood vessel is smaller relatively to the diameter, and, accordingly, the inclination angle of the helix against the circumferential direction Y is smaller (the smooth muscle cells are more parallel to the circumferential direction Y). Therefore, the inclination angle of the linear coating parts 24 relative to the circumferential direction Y is preferably set according to the blood vessel to which the stent 10 is to be applied, and, in some cases, the linear coating parts 24 may not necessarily be inclined (may be parallel to the circumferential direction Y).

In a blood vessel, vascular endothelial cells are disposed on the vascular smooth muscle cells arranged helically. In the stent according to this embodiment, the linear coating parts 24 having the cell adhesion ability are so provided as to extend along the circumferential direction of the stent body 12 when the stent body 12 is expanded. Therefore, the vascular smooth muscle cells are proliferated on the linear coating parts 24 while being arranged in the circumferential direction Y. After their proliferation on the linear coating parts 24, the vascular smooth muscle cells are further proliferated in such a manner as to fill up the gaps between the adjacent linear coating parts 24. Finally, the vascular endothelial cells are further proliferated on the vascular smooth muscle cells, whereby the inner surface of the stent body 12 is entirely coated with the vascular endothelial cells. Thus, by preliminarily restricting the ranges in which the vascular smooth muscle cells are proliferated, the vascular smooth muscle cells can be proliferated in a desirable form similar to the intrinsic form thereof. As a result, quicker cell restoration can be realized, and the onset of late stent thrombosis or restenosis can be restrained.

The width W of each of the linear coating parts 24 is preferably such a size that the vascular smooth muscle cells adhered will be liable to be arranged helically. The width W of each of the linear coating parts 24 is preferably not more than 50 µm, more preferably not more than 25 µm, and further preferably not more than 12.5 µm.

After the proliferation of the vascular smooth muscle cells on the linear coating parts 24, the vascular smooth muscle cells are further proliferated in such a manner as to fill up the gaps between the adjacent linear coating parts 24. In view of this, the spacing S between the adjacent liner coating parts 24 is preferably such a size that the vascular smooth muscle cells to be proliferated will be liable to be proliferated helically. The spacing S between the adjacent linear coating parts 24 is preferably not more than 50 µm, more preferably not more than 25 µm, and further preferably not more than 12.5 µm.

In addition, as shown in FIG. 9, which illustrates a modification of the stent 20 according to the second embodiment, the linear coating parts 24 may be so provided as to fill up a plurality of grooves 25 formed in the inner surface of the stent body 12. This configuration makes it possible to further lower the risk of breakage of the coating layer 23 at the time of an operation of mounting the stent onto a balloon or a balloon-inflating operation. Besides, this configuration makes it possible to further lessen the risk of breakage of the coating layer 23, without providing the linear coating parts 24 in the state of solid phase on the stent body 12.

### Examples

Now, the present invention will be described more in detail below referring to examples, but the invention is not to be restricted to or by the examples.

### Reference Example

A coated substrate 30 wherein BD^{™} PuraMatrix^{™} Peptide Hydrogel (registered trademark) was provided in a stripe pattern on a substrate (Co-Cr alloy) (supplied by HIGHTEMPMETALS; product code: L605) to form a plurality of linear coating parts 34, as shown in FIGS. 10 and 11, and a substrate (untreated substrate) wherein the same blank material as the above-mentioned substrate was not provided with any coating part, were put to comparative experiments, using cell culture. The width W of each of the linear coating parts 34 was 200 µm, and the spacing D between the adjacent linear coating parts 34 was 200 µm.

For the cell culture, normal human aorta-derived vascular endothelial cells were used. In the cell culture, the cells were incubated in a CO₂ incubator (5% CO₂, 37°C) by using a 10% fetal bovine serum (FBS)-added CSC-C culture medium and replacing the culture medium twice a week. The normal human aorta-derived vascular endothelial cells were seeded at 5×104cells/well in a 12-well plate in which the coated substrate 30 and the untreated substrate were placed, and the culture was conducted for 72 hours.

After incubation for 72 hours, the substrates were transferred into a new 12-well plate, to which a culture medium with a viable cell count measuring reagent SF added in a concentration of 10% was added in an additional amount of 1 ml per well. After incubation for three hours under the conditions of 37°C and 5% CO₂, absorbance of each well was measured by use of a microplate reader, whereby the viable cell count was measured.

Consequently, as shown in FIG. 12, it was confirmed that the viable cell count on the coated substrate 30 was greater than that on the untreated substrate. From this result, it was verified that the BD^{™} PuraMatrix^{™} Peptide Hydrogel (registered trademark) contained in the coating parts of the coated substrate promoted proliferation of vascular endothelial cells.

### Working Example

A stent was produced by providing linear coating parts formed of BD^{™} PuraMatrix^{™} Peptide Hydrogel (registered trademark) on an inner surface of a cylindrical stent body made of a metal (Co-Cr alloy) and having an outside diameter of 3.0 mm and a length of 15 mm. The extending direction of the linear coating parts of the stent was coincident with the longitudinal direction X of the stent, the width W of each of the linear coating parts was 50 µm, and the spacing S between the adjacent linear coating parts was 50 µm.

The stent was put indwelling in a coronary artery of a rabbit. After the stent was left indwelling there for 14 days, autopsy was conducted to take out the stent from the rabbit, and was bisected in the major axis direction by use of a pair of scissors. Vascular endothelial cells coating the lumen of the stent thus bisected was observed under scanning electron microscope (SEM).

### Comparative Example 1

The same observation as in Working Example was conducted, using a stent which was the same as that in Working Example except that the BD^{™} PuraMatrix^{™} Peptide Hydrogel (registered trademark) was provided on the whole area of the inner surface of the stent body.

### Comparative Example 2

The same observation as in Working Example was conducted, using a stent which was the same as that in Working Example except that the BD^{™} PuraMatrix^{™} Peptide Hydrogel (registered trademark) was not provided on the stent body.

Consequently, it was observed that, of the stents put indwelling, the stent of Working Example provided with the linear coating parts was coated with vascular endothelial cells most. From this result, it was verified that the BD^{™} PuraMatrix^{™} Peptide Hydrogel (registered trademark) used for coating and the structure of the linear coating parts promote the proliferation of vascular endothelial cells.

Incidentally, the present invention is not restricted to the above-described embodiments, and various alterations are possible within the scope of the claims. For instance, other layer(s) than the coating layer having the cell adhesion ability may be provided on the stent, and the outer surface of the stent body which comes into contact with a living body tissue may be provided thereon with a layer of a biologically active agent having a restenosis-restraining effect. In addition, the linear coating parts may not necessarily be provided over the whole area of the inner surface of the stent body. Besides, while the first embodiment is for promoting the proliferation of vascular endothelial cells whereas the second embodiment is for promoting the proliferation of vascular smooth muscle cells, the present invention is not limited to these purposes, and can be applied with appropriate design modifications according to the cells in the site in which the stent is to be placed.

Furthermore, the present application is based on Japanese Patent Application No. 2010-076616, filed on March 30, 2010.

### Description of Reference Characters

- 10, 20: Stent
- 11: Annular part
- 12: Stent body
- 13, 23: Coating layer
- 14, 24: Linear coating part
- 15, 25: Groove
- S, D: Spacing
- W: Width
- X: Longitudinal direction
- Y: Circumferential direction

## Claims

1. A stent (10, 20) comprising:
a cylindrical stent body (12) which has openings at both ends and extends along a longitudinal direction (X) between the openings at the both ends; and
a coating layer (13, 23) which is provided on an inner surface of the stent body (12) and contains a substance having a cell adhesion ability to promote adhesion of cells,
wherein the coating layer (13, 23) is formed by arranging a plurality of linear coating parts (14, 24), each extending linearly, in a striped pattern,
**characterized in that**
the linear coating parts (14, 24) are so provided as to extend along the longitudinal direction (X) of the stent body (12) when the stent body (12) is expanded.

2. A stent (10, 20) comprising:
a cylindrical stent body (12) which has openings at both ends and extends along a longitudinal direction (X) between the openings at the both ends; and
a coating layer (13, 23) which is provided on an inner surface of the stent body (12) and contains a substance having a cell adhesion ability to promote adhesion of cells,
wherein the coating layer (13, 23) is formed by arranging a plurality of linear coating parts (14, 24), each extending linearly, in a striped pattern,
**characterized in that**
the linear coating parts (14, 24) are so provided as to extend along a circumferential direction (Y) of the stent body (12) when the stent body (12) is expanded.

3. The stent (10, 20) according to any of claims 1 or 2, wherein the linear coating parts (14, 24) are provided in the state of filling grooves (15, 25) formed in the inner surface of the stent body (12).

4. The stent (10, 20) according to any of claims 1 to 3, wherein the substance having the cell adhesion ability is a synthetic peptide.

## Patentansprüche

1. Stent (10, 20), welcher umfasst:
einen zylindrischen Stentkörper (12), welcher an beiden Enden Öffnungen hat und sich entlang einer longitudinalen Richtung (X) zwischen diesen Öffnungen erstreckt; und
eine Beschichtung (13, 23), welche auf einer Innenfläche des Stentkörpers (12) bereitgestellt ist und eine zelladhäsionsfähige Substanz beinhaltet, um Adhäsion von Zellen zu fördern,
wobei die Beschichtung (13, 23) durch Anordnen einer Vielzahl von sich jeweils linear erstreckenden linearen Beschichtungsteilen (14, 24) in einem Streifenmuster ausgebildet ist,
**dadurch gekennzeichnet, dass**
die linearen Beschichtungsteile (14, 24) so bereitgestellt sind, dass sie sich entlang der longitudinalen Richtung (X) des Stentkörpers (12) erstrecken, wenn der Stentkörper (12) ausgedehnt wird.

2. Stent (10, 20), welcher umfasst:
einen zylindrischen Stentkörper (12), welcher an beiden Enden Öffnungen hat und sich entlang einer longitudinalen Richtung (X) zwischen diesen Öffnungen erstreckt; und
eine Beschichtung (13, 23), welche auf einer Innenfläche des Stentkörpers (12) bereitgestellt ist und eine zelladhäsionsfähige Substanz beinhaltet, um Adhäsion von Zellen zu fördern,
wobei die Beschichtung (13, 23) durch Anordnen einer Vielzahl von sich jeweils linear erstreckenden linearen Beschichtungsteilen (14, 24) in einem Streifenmuster ausgebildet ist,
**dadurch gekennzeichnet, dass**
die linearen Beschichtungsteile (14, 24) so bereitgestellt sind, dass sie sich entlang der Umfangsrichtung (Y) des Stentkörpers (12) erstrecken, wenn der Stentkörper (12) ausgedehnt wird.

3. Stent (10, 20) nach Anspruch 1 oder 2, wobei die linearen Beschichtungsteile (14, 24) derart bereitgestellt sind, dass sie Nuten (15, 25) ausfüllen, welche in der Innenfläche des Stentkörpers (12) gebildet sind.

4. Stent (10, 20) nach einem der Ansprüche 1 bis 3, wobei die zelladhäsionsfähige Substanz ein synthetisches Peptid ist.

## Revendications

1. Stent (10, 20) comprenant :
un corps de stent cylindrique (12) qui comporte des ouvertures au niveau des deux extrémités et s'étend suivant une direction longitudinale (X) entre les ouvertures au niveau des deux extrémités ; et
une couche de revêtement (13, 23) qui est prévue sur une surface interne du corps de stent (12) et contient une substance présentant une capacité d'adhésion de cellules permettant de favoriser l'adhésion de cellules,
dans lequel la couche de revêtement (13, 23) est formée en disposant une pluralité de parties de revêtement linéaires (14, 24), chacune s'étendant de façon linéaire, dans un motif rayé,
**caractérisé en ce que**
les parties de revêtement linéaires (14, 24) sont disposées de manière à s'étendre dans la direction longitudinale (X) du corps de stent (12) lorsque le corps de stent (12) est déployé.

2. Stent (10, 20) comprenant :
un corps de stent cylindrique (12) qui comporte des ouvertures au niveau des deux extrémités et s'étend suivant une direction longitudinale (X) entre les ouvertures au niveau des deux extrémités ; et
une couche de revêtement (13, 23) qui est prévue sur une surface interne du corps de stent (12) et contient une substance présentant une capacité d'adhésion de cellules permettant de favoriser l'adhésion de cellules,
dans lequel la couche de revêtement (13, 23) est formée en disposant une pluralité de parties de revêtement linéaires (14, 24), chacune s'étendant de façon linéaire, dans un motif rayé,
**caractérisé en ce que**
les parties de revêtement linéaires (14, 24) sont disposées de manière à s'étendre suivant une direction circonférentielle (Y) du corps de stent (12) lorsque le corps de stent (12) est déployé.

3. Stent (10, 20) selon l'une quelconque des revendications 1 ou 2, dans lequel les parties de revêtement linéaires (14, 24) sont disposées de manière à remplir des rainures (15, 25) formées dans la surface interne du corps de stent (12).

4. Stent (10, 20) selon l'une quelconque des revendications 1 à 3, dans lequel la substance présentant la capacité d'adhésion de cellules est un peptide synthétique.
